Europäisches Patentamt

European Patent Office

Office européen des brevets

(19)

(11) Veröffentlichungsnummer: **0 309 841**
**A2**

## (12) EUROPÄISCHE PATENTANMELDUNG

(21) Anmeldenummer: 88115274.8

(22) Anmeldetag: 17.09.88

(51) Int. Cl.4: **C07K 5/00 , A61K 37/64**

(30) Priorität: 02.10.87 DE 3733296

(43) Veröffentlichungstag der Anmeldung:
05.04.89 Patentblatt 89/14

(84) Benannte Vertragsstaaten:
**AT BE CH DE ES FR GB IT LI NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Raddatz, Peter, Dr.**
**Grafenstrasse 37**
**D-6100 Darmstadt(DE)**
Erfinder: **Schmittges, Claus J., Dr.**
**Karolinger Strasse 5**
**D-6114 Gross-Umstadt(DE)**
Erfinder: **Minck, Klaus-Otto, Dr.**
**Büchestrasse 8**
**D-6104 Ober-Ramstadt(DE)**

(54) **Aminosäurederivate.**

(57) Neue Aminosäurederivate der Formel I

$$X\text{-}Z\text{-}NR^3\text{-}CHR^4\text{-}CR^5\text{-}(CHR^6)_n\text{-}E$$

worin

X, Z, E, $R^3$, $R^4$, $R^5$, $R^6$, und n die in Patentanspruch
1 angegebene Bedeutung haben,
sowie ihre Salze hemmen die Aktivität des menschlichen Plasmarenins.

EP 0 309 841 A2

## Aminosäurederivate

Die Erfindung betrifft neue Aminosäurederivate der Formel I

X-Z-NR³-CHR⁴-CR⁵-(CHR⁶)ₙ-E    I

worin

X    H, $R^1$-O-$C_mH_{2m}$-CO-, $R^1$-$C_mH_{2m}$-O-CO-, $R^1$-$C_mH_{2m}$-CO-, $R^1$-SO₂-, ($R^1$-$C_mH_{2m}$)-L($R^2$-$C_pH_{2p}$)-$C_rH_{2r}$-CO-, $R^1$-(NHCH₂CH₂)$_m$-NH-CH₂CO-, 9-Fluorenyl-$C_mH_{2m}$-O-CO-, [$R^1$-$C_mH_{2m}$-(T)$_x$-V-$C_tH_{2t}$]-L-($R^2$-$C_pH_{2p}$)-$C_rH_{2r}$-CO-oder $A_3N^{\oplus}$-$C_mH_{2m}$-CO- An$^{\ominus}$,

Z    1 bis 4 peptidartig miteinander verbundene Aminosäurereste ausgewählt aus der Gruppe bestehend aus Abu, Ada, Ala, ß-Ala, Arg, Asn, Asp, Bia, Cal, Dab, Ftr, Gln, Glu, Gly, His, Hph, N(im)-A-His, Ile, Leu, tert.-Leu, Lys, Met, αNal, ßNal, Nbg, Nle, Nva, Orn, Phe, Pro, Pyr, Ser, Thr, Tia, Tic, Trp, Tyr, O-A-Tyr und Val,

E    S-$R^7$, SO-$R^7$, SO₂-$R^7$, SO₂-O$R^7$ oder SO₂-N$R^7R^8$,

   $R^1$, $R^2$, $R^4$, $R^7$ und $R^8$    jeweils H, A, Ar, Aralkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^3$    H oder A,

$R^5$    jeweils (H, OH), (H, NH₂) oder = O,

$R^6$    H, A oder Alkenyl mit 2-8 C-Atomen,

m, p, r und t    jeweils 0, 1, 2, 3, 4 oder 5,

n    1 oder 2,

x    0 oder 1,

L    CH oder N,

T    O oder NH,

V    CO oder SO₂,

An$^{\ominus}$    ein Äquivalent eines Anions,

Ar    unsubstituiertes oder ein- oder mehrfach durch A, AO, Hal, CF₃, HO, Hydroxyalkyl mit 1-8 C-Atomen, H₂N und/oder Aminoalkyl mit 1-8 C-Atomen substituiertes Phenyl oder unsubstituiertes Naphthyl,

Het    einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, CF₃, HO, O₂N, Carbonylsauerstoff, H₂N, HAN, A₂N, AcNH, AS, ASO, ASO₂, AOOC, CN, H₂NCO, H₂NSO₂, ASO₂NH, Ar, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

Hal    F, Cl, Br oder J,

Ac    A-CO-, Ar-CO- oder A-NH-CO- und

A    Alkyl mit 1-8 C-Atomen bedeuten, worin ferner an Stelle einer oder mehrerer -NH-CO-Gruppen auch eine oder mehrere -NA-CO-Gruppen stehen können, sowie deren Salze.

Ähnliche Verbindungen sind aus der EP-A-163237 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich höhere Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor- und nachstehend aufgeführten Abkürzungen von Aminosäureresten stehen für die Reste -NR″-R′-CO-, in der Regel -NH-CHR-CO- (worin R′, R′ und R″ die für jede Aminosäure bekannte spezifische Bedeutung haben) folgender Aminosäuren:

| | |
|---|---|
| Abu | 2-Aminobuttersäure |
| Ada | 3-(1-Adamantyl)-alanin |
| Ala | Alanin |
| ß-Ala | ß-Alanin |
| Arg | Arginin |
| Asn | Asparagin |
| Asp | Asparaginsäure |
| Bia | 3-(2-Benzimidazolyl)-alanin |
| Cal | 3-Cyclohexylalanin |

Dab    2,4-Diaminobuttersäure
Ftr    N(ind)-Formyl-tryptophan
Gln    Glutamin
Glu    Glutaminsäure
Gly    Glycin
His    Histidin
Hph    Homo-phenylalanin (2-Amino-4-phenylbuttersäure)
N(im)-A-His    in 1- oder 3-Stellung des Imidazolrings durch A substituiertes Histidin
Ile    Isoleucin
Leu    Leucin
tert.-Leu    tert.-Leucin
Lys    Lysin
Mal    3-(p-Methoxyphenyl)-alanin
Met    Methionin
$\alpha$Nal    3-($\alpha$-Naphthyl)-alanin
$\beta$Nal    3-($\beta$-Naphthyl)-alanin
Nbg    (2-Norbornyl)-glycin
Nle    Norleucin
Nva    Norvalin
Orn    Ornithin
Phe    Phenylalanin
Pro    Prolin
Pyr    3-(Pyridyl)-alanin, z.B. 3-Pyr = 3-(3-Pyridyl)-alanin
Ser    Serin
Thr    Threonin
Tia    3-(Thienyl)-alanin, z.B. 2-Tia = 3-(2-Thienyl)-alanin
Tic    1,2,3,4-Tetrahydroisochinolin-1-carbonsäure
Trp    Tryptophan
Tyr    Tyrosin
O-A-Tyr    O-Alkyl-tyrosin
Val    Valin.

Ferner bedeutet nachstehend:

BOC    tert.-Butoxycarbonyl
imi-BOM    Benzyloxymethyl in 1-Stellung des Imidazolrings
CBZ    Benzyloxycarbonyl
DNP    2,4-Dinitrophenyl
imi-DNP    2,4-Dinitrophenyl in 1-Stellung des Imidazolrings
FMOC    9-Fluorenylmethoxycarbonyl
IPOC    Isopropoxycarbonyl
MC    Morpholinocarbonyl
POA    Phenoxyacetyl
PyOC    2-(2-Pyridyl)-ethoxy-carbonyl
DCCI    Dicyclohexylcarbodiimid
HOBt    1-Hydroxybenzotriazol.

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung sind die Aminosäurederivate der Formel I sowie deren Salze, ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

$$X-G^1-OH \qquad II$$

worin $G^1$:

(a) $-Z^1-$
(b) $-Z-$ bedeutet und
X    die angegebene Bedeutung hat,
oder eines ihrer reaktionsfähigen Derivate mit einer Verbindung der Formel III

$$H-G^2 \qquad III$$

worin
$G^2$

(a) $-Z^2-NR^3-CHR^4-CR^5-(CHR^6)_n-E$,
(b) $-NR^3-CHR^4-CR^5-(CHR^6)_n-E$ und
$Z^1 + Z^2$    zusammen Z bedeuten und
$R^3$, $R^4$, $R^5$, $R^6$, n und E    die angegebenen Bedeutungen haben,

oder einem ihrer reaktionsfähigen Derivate umsetzt,
und/oder daß man gegebenenfalls zur Herstellung von Verbindungen der Formel I, worin E SO-$R^7$ oder -SO$_2$-$R^7$ bedeutet, ein Sulfid der Formel I, worin E S-$R^7$ bedeutet, mit einem Oxydationsmittel behandelt, und/oder daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine Ketogruppe zu einer CHOH-Gruppe reduziert oder zu einer CH(NH$_2$)-Gruppe reduktiv aminiert und/oder eine Verbindung der Formel I durch Behandeln mit einer Säure in eines ihrer Salze überführt.

Vor- und nachstehend haben die Reste bzw. Parameter X, Z, E, $R^1$ bis $R^8$, m, n, p, r, t, x, L, T, An, Ar, Het, Hal, Ac, A, $G^1$, $G^2$, $Z^1$ und $Z^2$ die bei den Formeln I, II oder III angegebenen Bedeutungen, falls nicht ausdrücklich etwas anderes angegeben ist. Falls zwei Reste $R^6$ in einer Verbindung

der Formel I vorhanden sind, können sie gleich oder voneinander verschieden sein.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z. B. 1-, 2- oder 3-Methylcyclopentyl, 1-. 2-, 3- oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1- oder 2-Adamantyl.

Hal bedeutet vorzugsweise F, Cl oder Br, aber auch J.

Ac bedeutet vorzugsweise A-CO- wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m-oder p-Hydroxyphenyl, o-, m- oder p-Hydroxymethylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m-oder p-Aminomethylphenyl, 1- oder 2-Naphthyl.

Dementsprechend bedeutet Ar-alkyl vorzugsweise Benzyl, 1-oder 2-Phenylethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m-oder p-Hydroxybenzyl, o-, m- oder p-Hydroxymethylbenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, 1- oder 2-Naphthylmethyl.

Het ist vorzugsweise 2- oder 3-Furyl, 2- oder 3-Thienyl, 1-, 2- oder 3-Pyrryl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 2.1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6 oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7-, oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6-, 7- oder 8-Chinazolyl. Die heterocyclischen Reste konnen auch teilweise oder vollständig hydriert sein. Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, Tetrahydro-2- oder -3-thienyl, 2,3-Dihydro-1-, -2-, -3-, -4- oder -5-pyrryl, 2,5-Dihydro-1-, -2-, -3-, -4- oder -5-pyrryl, 1-, 2- oder 3-Pyrrolidinyl, Tetrahydro-1-, -2-oder -4-imidazolyl, 2,3-Dihydro-1-, -2-, -3-, -4- oder 5-pyrazolyl, 2,5-Dihydro-1-, -2-, -3-, -4- oder 5-pyrazolyl, Tetrahydro-1-, -3-, oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidyl, 1-, 2- oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4-oder 5-Methyl-3-thienyl, 3-Methyl-5-tert.-butyl-2-thienyl, 5-Chlor-2-thienyl, 5-Phenyl-2- oder -3-thienyl, 1-, 3-, 4 oder 5-Methyl-2-pyrryl, 1-Methyl-4- oder -5-nitro-2-pyrryl, 3,5-Dimethyl-4-ethyl-2-pyrryl, 4-Methyl-5-pyrazolyl, 4-oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 5- oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl.

$R^1$ und $R^2$ bedeuten vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cyclohexyl, Methylcyclohexyl wie trans-4-Methylcyclohexyl, tert. Butylcyclohexyl wie trans-4-tert.-Butylcyclohexyl, Phenyl oder Benzyl.

$R^3$, bedeutet vorzugsweise H oder Methyl, ferner bevorzugt Ethyl, Propyl, Isopropyl, Butyl oder Isobutyl.

$R^4$ bedeutet vorzugsweise Isobutyl oder Cyclohexylmethyl, ferner bevorzugt A, insbesondere Methyl, Ethyl, Propyl, Isopropyl, Butyl, sek.-Butyl, Pentyl, Isopentyl (3-Methylbutyl) oder 2-Methylbutyl, Phenyl, Benzyl, p-Chlorbenzyl, 1- oder 2-Naphthylmethyl, 2-Phenylethyl, 3-Phenylpropyl, 2-Cyclohexylethyl, 1- oder 2-Dekalylmethyl, Bicyclo-[2,2,1]heptyl-2-methyl oder 6,6-Dimethylbicyclo-[3,1,1]heptyl-2-methyl.

$R^5$ ist vorzugsweise (H, OH).

$R^6$ bedeutet vorzugsweise H oder Isopropyl, ferner bevorzugt Methyl, Ethyl, Propyl, Butyl, Isobutyl oder sek.-Butyl.

Falls E S-$R^7$, SO-$R^7$ oder (vorzugsweise) SO$_2$-$R^7$ bedeutet, ist $R^7$ vorzugsweise A, Ar oder Het, insbesondere Phenyl oder ein gesättigter 5- oder 6-gliedriger heterocyclischer Rest wie Pyrrolidino, Piperidino, Morpholino, Piperazino, 4-A-piperazino (z.B. 4-Methylpiperazino), 4-Ar-piperazino (z.B. 4-Phenylpiperazino), 4-(Hydroxyalkyl)-piperazino [z.B. 4-(2-Hydroxyethyl)-piperazino]. Falls E SO$_2$-OR$^7$ bedeutet, ist $R^7$ vorzugsweise A oder Ar. Falls E

SO$_2$-NR$^7$R$^8$ bedeutet, sind $R^7$ und $R^8$ vorzugsweise unabhängig voneinander jeweils H, A, Ar oder Ar-alkyl. Dementsprechend ist E bevorzugt Morpholinosulfonyl, ferner Pyrrolidinosulfonyl, Piperidinosulfonyl, Piperazinosulfonyl, 4-Methylpiperazinosulfonyl, Phenylsulfonyl, Sulfamoyl, N-Methylsulfamoyl, N-Ethylsulfamoyl, N-Propylsulfamoyl, N-Isopropylsulfamoyl, N-Butylsulfamoyl, N-Isobutylsulfamoyl, N-sek.-Butylsulfamoyl, N-Pentylsulfamoyl, N-Isopentylsulfamoyl, N-Hexylsulfamoyl, N,N-Dimethylsulfamoyl, N,N-Diethylsulfamoyl, N.N-Dipropylsulfamoyl, N,N-Dibutylsulfamoyl, N-Benzyl-N-methyl-sulfamoyl, N-Benzyl-N-ethyl-sulfamoyl, N-Benzyl-N-propyl-sulfamoyl, N-Benzyl-N-butyl-sulfamoyl, weiterhin z.B. Morpholinosulfinyl, Pyrrolidinosulfinyl, Piperidinosulfinyl, Piperazinosulfinyl, Phenylsulfinyl, Morpholinothio, Phenylthio.

L ist vorzugsweise CH. T ist vorzugsweise NH. An ist vorzugsweise Cl, Br, HCOO oder CH$_3$COO.

Die Parameter m, p, r und t sind vorzugsweise 0, 1 oder 2; n ist vorzugsweise 2; x ist vorzugsweise 1.

X bedeutet vorzugsweise H, POA, Alkoxycarbonyl wie IPOC oder BOC, CBZ, Alkanoyl wie Acetyl, Propionyl, Butyryl Isobutyryl, Isovaleryl oder 3,3-Dimethylbutyryl, Cycloalkylcarbonyl wie Cyclopentylcarbonyl oder Cyclohexylcarbonyl, Aroyl wie Benzoyl, Arylalkanoyl wie Phenylacetyl, 2- oder 3-Phenylpropionyl, 4-Phenylbutyryl, 2-Benzylheptanoyl, 2-Benzyl-3-phenylpropionyl, 2-Benzyl-4-phenylbutyryl, 2-(2-Phenylethyl)-4-phenylbutyryl, 2-(2-Naphthylmethyl)-4-phenylbutyryl, Bis-(1-naphthylmethyl)-acetyl), 2- oder 3-o-, -m- oder -p-Fluorphenylpropionyl, 2- oder 3-o-, -m- oder -p-Chlorphenylpropionyl, Cycloalkylalkanoyl wie Cyclohexylacetyl, 2-oder 3-Cyclohexylpropionyl, Hetalkanoyl wie MC, Morpholinoacetyl, 4-(2-, 4-(3- oder 4-(4-Pyridyl)-butyryl, PyOC, Amino-alkanoyl wie 6-Aminohexanoyl, 4-Dimethylaminobutyryl, 6-Dimethylaminohexanoyl, FMOC. Besonders bevorzugte Reste X sind BOC und Bis-(1-naphtylmethyl)-acetyl, ferner POA, 4-Phenylbutyryl, 2-Benzyl-3-phenylpropionyl, 2-Benzyl-4-phenylbutyryl, 2-(2-Phenylethyl)-4-phenylbutyryl, 2-(2-Naphthylmethyl)-4-phenylbutyryl und CBZ.

Z bedeutet vorzugsweise 2, aber auch 1, weiterhin 3 oder 4 peptidartig miteinander verbundene Aminosäurereste, insbesondere eine der Gruppen His, Phe-His oder Phe-Gly, ferner bevorzugt die Gruppen Abu, Ada, Asn, Bia, Cal, Gln, N-(im)-Methyl-His, Leu, αNal, βNal, Nle, Phe, Trp, Tyr, Abu-His, Ada-His, Ala-His, Ala-Phe, Arg-His, Asn-His, Bia-His, Cal-His, Dab-His, Glu-His, His-His, Hph-His, Ile-His, Leu-His, tert.-Leu-His, Lys-His, Met-His, αNal-His, βNal-His, Nbg-His, Nle-His, (N-Me-His)-His, (N-Me-Phe)-His, Orn-His, Phe-Abu, Phe-Ada, Phe-Ala, Phe-βAla, Phe-Arg, Phe-Asn, Phe-Bia, Phe-Cal, Phe-Dab, Phe-Gln, Phe-Glu, Phe-(N-im-Methyl-His), Phe-Ile, Phe-Leu, Phe-tert.-Leu, Phe-Lys, Phe-Met, Phe-α-Nal, Phe-βNal, Phe-Nbg, Phe-Nle, Phe-(N-Me-His), Phe-(N-Me-Phe), Phe-Orn, Phe-Phe, Phe-Pro, Phe-(2-Pyr), Phe-(3-Pyr), Phe-(4-Pyr), Phe-Ser, Phe-Thr, Phe-Tic, Phe-Trp, Phe-Tyr, Phe-Val, Pro-His, Ser-His, Thr-His, Tic-His, Trp-His, Tyr-His, (O-Me-Tyr)-His, Val-His, ferner Ada-Phe-His, Pro-Ala-His, Pro-Ala-Phe, Pro-Phe-Ala, Pro-Phe-His, Pro-Phe-Phe, Ftr-Pro-Phe-His, His-Pro-Ala-His, His-Pro-Ala-Phe, His-Pro-Phe-Ala, His-Pro-Phe-Phe, weiterhin Pro-Abu-His, Pro-Ada-His, Pro-Arg-His, Pro-Asn-His, Pro-Bia-His, Pro-Dab-His, Pro-Glu-His, Pro-His-His, Pro-Ile-His, Pro-Leu-His, Pro-tert.-Leu-His, Pro-Lys-His, Pro-Met-His, Pro-Nbg-His, Pro-Nle-His, Pro-(N-Me-His)-His, Pro-(N-Me-Phe)-His, Pro-Orn-His, Pro-Phe-Abu, Pro-Phe-Ada, Pro-Phe-Arg, Pro-Phe-Asn, Pro-Phe-Bia, Pro-Phe-Dab, Pro-Phe-Gln, Pro-Phe-Glu, Pro-Phe-(N-im-Methyl-His), Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser-His, Pro-Thr-His, Pro-Tic-His, Pro-Trp-His, Pro-Tyr-His, Pro-Val-His, His-Pro-Abu-His, His-Pro-Ada-His, His-Pro-Arg-His, His-Pro-Asn-His, His-Pro-Bia-His, His-Pro-Dab-His, His-Pro-Glu-His, His-Pro-His-His, His-Pro-Ile-His, His-Pro-Leu-His, His-Pro-tert.-Leu-His, His-Pro-Lys-His, His-Pro-Met-His, His-Pro-Nbg-His, His-Pro-Nle-His, His-Pro-(N-Me-His)-His, His-Pro-(N-Me-Phe)-His, His-Pro-Orn-His, His-Pro-Phe-Abu, His-Pro-Phe-Ada, His-Pro-Phe-Arg, His-Pro-Phe-Asn, His-Pro-Phe-Bia, His-Pro-Phe-Dab, His-Pro-Phe-Gln, His-Pro-Phe-Glu, His-Pro-Phe-His, His-Pro-Phe(N-im-Methyl-His), His-Pro-Phe-Ile, His-Pro-Phe-Leu, His-Pro-Phe-tert.-Leu, His-Pro-Phe-Lys, His-Pro-Phe-Met, His-Pro-Phe-Nbg, His-Pro-Phe-Nle, His-Pro-Phe-(N-Me-His), His-Pro-Phe-(N-Me-Phe), His-Pro-Phe-Orn, His-Pro-Phe-Pro, His-Pro-Phe-Ser, His-Pro-Phe-Thr, His-Pro-Phe-Tic, His-Pro-Phe-Trp, His-Pro-Phe-Tyr, His-Pro-Phe-Val, His-Pro-Pro-His, His-Pro-Ser-His, His-Pro-Thr-His, His-Pro-Tic-His, His-Pro-Trp-His, His-Pro-Tyr-His, His-Pro-Val-His.

Die Gruppe -NR$^3$-CHR$^4$-CR$^5$-(CHR$^6$)$_n$- bedeutet vorzugsweise -NH-CHR$^4$-CHOH-CH$_2$-, insbesondere -NH-CH(Cyclohexylmethyl)-CHOH-CH$_2$- ferner -NH-CH(CH$_2$CH$_2$-Cyclohexyl)-CHOH-CH$_2$-, -NH-CH-(Isobutyl)-CHOH-CH$_2$- oder -NH-CH(Benzyl)-CHOH-CH$_2$, ferner bevorzugt -NH-CHR$^4$-CH(NH$_2$)-CH$_2$-, insbesondere -NH-CH(Cyclohexylmethyl)-CH(NH$_2$)-CH$_2$-, -NH-CH(CH$_2$CH$_2$-Cyclohexyl)-CH-(NH$_2$)-CH$_2$-, -NH-CH(Isobutyl)-CH(NH$_2$)-CH$_2$-oder -NH-CH(Benzyl)-CH(NH$_2$)-CH$_2$-, weiterhin bevorzugt -NH-CHR$^4$-CHOH-CH$_2$-CHA-, insbesondere -NH-CH(Cyclohexylmethyl)-CHOH-CH$_2$-CH(Isopropyl)-.

Diese Gruppe besitzt in der Regel zwei chirale Zentren. Die Verbindungen der Formel I können daher in verschiedenen - optisch-inaktiven oder optisch-aktiven - Formen vorkommen. Die Formel I umschließt alle diese Formen. Der Substituent X-Z-NR$^3$- liegt vorzugsweise in Form des S-Enantiomeren vor.

Die Gruppe E ist vorzugsweise SO$_2$-R$^7$, bevorzugt SO$_2$-Het, insbesondere SO$_2$-Morpholino, ferner bevorzugt SO$_2$-Pyrrolidino, SO$_2$-Piperidino oder SO$_2$-Piperazino; weiterhin ist E vorzugsweise SO$_2$-NR$^7$R$^8$, insbesondere SO$_2$-N(A)$_2$ wie SO$_2$-N(CH$_3$)$_2$; SO$_2$-NHA wie SO$_2$-NH-C$_4$H$_9$, SO$_2$-NH-Isopentyl; SO$_2$-NAAr-alkyl wie SO$_2$-N(CH$_2$C$_6$H$_5$)C$_4$H$_9$.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen, worin jedoch

in Ia

X    BOC, Isovaleryl, 3,3-Dimethylbutyryl, Bis-(1-napththylmethyl)-acetyl, 2-Benyzl-4-phenyl-butyryl, POA, 2-(1-Naphthylmethyl)-3-N-isopropylcarbamoyl-propionyl, 2-Benzyl-3-MC-propionyl, N-Benzyl-N-butyl-carbamoyl, 6-Aminohexanoyl, Morpholinoacetyl, PyOC, IPOC, MC, 4-(4-Pyridyl)-butyryl, 4-Di-methylamino-butyryl, 6-Dimethylaminohexanoyl, 4-Trimethyl-ammoniumbutyryl-formiat, 6-Trimethylammonium-hexanoyl-formiat oder CBZ bedeutet;

in Ib

X    BOC oder Bis-(1-naphthylmethyl)-acetyl bedeutet;

in Ic

X    BOC bedeutet;

in Id

Z    His, Bia-His, Cal-His, Hph-His, αNal-His, Nle-His, Phe-Abu, Phe-Ala, Phe-βAla, Phe-Asn, Phe-Gln, Phe-Gly, Phe-His, Phe-Leu, Phe-Met, Phe-Nle, Phe-(N-Me-His), Phe-(3-Pyr), Trp-His, (O-Me-Tyr)-His, Pro-Phe-His, Pro-Phe-N(Me)-His, His-Pro-Phe-His oder Ftr-Pro-Phe-His bedeutet;

in Ie

Z    His oder Phe-His bedeutet;

in If

R$^3$    H,

R$^4$    Isobutyl oder Cyclohexylmethyl,

R$^5$    OH,

R$^6$    H oder Isopropyl und

n    2 bedeuten;

in Ig

X    BOC oder Bis-(1-naphthylmethyl)-acetyl,

Z    His oder Phe-His,

R$^3$    H,

R$^4$    Isobutyl oder Cyclohexylmethyl,

R$^5$    OH,

$R^6$    H oder Isopropyl und

n    2 bedeuten.

Weiterhin sind bevorzugt Verbindungen, die den Formeln I sowie Ia bis Ig entsprechen, worin jedoch jeweils zusätzlich

    (a) E    $S-R^7$ bedeutet;

    (b) E    $SO-R^7$ bedeutet;

    (c) E    $SO_2-R^7$ bedeutet;

    (d) E    $SO_2-OR^7$ bedeutet;

    (e) E    $SO_2-NR^7R^8$ bedeutet;

    (f) E    $SO_2$-Het bedeutet;

    (g) E    $SO_2$-Morpholino, $SO_2$-Pyrrolidino, $SO_2$-Piperidino, $SO_2$-Piperazino, $SO_2$-NHA, $SO_2$N-$(A)_2$ oder $SO_2$NA-Aralkyl bedeutet;

    (h) E    $SO_2$-Morpholino bedeutet.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

So können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-$R^9$-His-Gruppe (worin $R^9$ eine Aminoschutzgruppe bedeutet, z. B. BOM oder DNP) enthalten, oder solche der Formel X-Z-$NR^3$-$CHR^4$-CH(NHR$^9$)-(CHR$^6$)$_n$-E.

Ferner sind Ausgangsstoffe bevorzugt, die an Stelle des H-Atoms einer Hydroxygruppe eine Hydroxyschutzgruppe tragen, z. B. solche der Formel X-Z-$NR^3$-$CHR^4$-CH(OR$^{10}$)-(CHR$^6$)$_n$-E, worin $R^{10}$ eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl- (z. B. 2,4-Dinitrophenyl), Aralkoxymethyl- (z. B. Benzyloxymethyl) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP, BOM, CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), Phosphorsäure-hexamethyltriamid (HMPT), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50$^\bullet$, vorzugsweise arbeitet man zwischen 15 und 30$^\bullet$ (Raumtemperatur).

Die Boc-Gruppe kann z. B. bevorzugt mit 40 %iger Trifluoressigsäure in Methylenchlorid oder mit etwa 3 bis 5 n HCl in Dioxan bei 15-30$^\bullet$ abgespalten werden, die FMOC-Gruppe mit einer etwa 5- bis 20%igen Lösung von Dimethylamin, Diethylamin oder Piperidin in DMF bei 15-30$^\bullet$. Eine Abspaltung der DNP-Gruppe gelingt z. B. auch mit einer etwa 3- bis 10%igen Lösung von 2-Mercaptoethanol in DMF/Wasser bei 15-30$^\bullet$.

Hydrogenolytisch entfernbare Schutzgruppen (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100$^\bullet$ und Drucken zwischen etwa 1 und 200 bar, bevorzugt bei 20-30$^\bullet$ und 1-10 bar durchgeführt. Eine Hydrogenolyse der CBZ-Gruppe gelingt z.B. gut an 5- bis 10%igem Pd-C in Methanol bei 20-30$^\bullet$.

Verbindungen der Formel I können auch durch direkte Peptidsynthese aus einer Carbonsäure- und einer Aminkomponente erhalten werden. Als Carbonsäurekomponenten eignen sich z. B. solche der Teilformel X-Z-OH, als Aminkomponenten solche der Teilformel H-NR$^3$-CHR$^4$-CR$^5$-(CHR$^6$)$_n$-E. Ferner kann die Peptidbindung innerhalb der Gruppe Z geknüpft werden, falls Z aus mindestens zwei der angegebenen Aminosäurereste besteht; dabei wird eine Carbonsäure der Formel X-Z$^1$-OH mit einem Amin der Formel H-Z$^2$-NR$^3$-CHR$^4$-CR$^5$-(CHR$^6$)$_n$-E umgesetzt, wobei Z$^1$ und Z$^2$ jeweils mindestens einen der bei der Definition von Z angegebenen Aminosäurereste bedeutet und Z$^1$ + Z$^2$ = Z ist. Dabei arbeitet man zweckmäßig nach üblichen Methoden der Peptid-Synthese, wie sie z. B. in Houben-Weyl, 1.c., Band, Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30$^\bullet$.

An Stelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von 1-Hydroxybenztriazol oder N-Hydroxysuccinimid.

Die Ausgangsstoffe der Formeln II und III sind teilweise bekannt. Sofern sie nicht bekannt sind, können sie nach bekannten Methoden, z.B. den oben oder in den Beispielen angegebenen Methoden oder in Analogie dazu, hergestellt werden.

Gewünschtenfalls kann ein Sulfid der Formel I, worin E S-R$^7$ bedeutet, zum entsprechenden Sulfoxid (I, E = SO-R$^7$) oder Sulfon (I, E = SO$_2$-R$^7$) oxidiert werden, z.B. mit Wasserstoffperoxid oder einer Persäure wie m-Chlorperoxybenzoesäure in einem inerten Lösungsmittel wie Chloroform bei Temperaturen zwischen etwa -10 bis +20$^\bullet$. Zur Herstellung des Sulfoxids verwendet man etwa stöchiometrische Mengen des Oxydationsmittels. Mit der zur Bildung des Sulfons berechneten oder einer überschüssigen Menge des Oxydationsmittels erhält man überwiegend das Sulfon.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die BOC-Gruppe abspalten.

In einer Verbindung der Formel I kann auch eine Ketogruppe zu einer CHOH-Gruppe reduziert werden, beispielsweise mit einem komplexen Metallhydrid wie NaBH4, das nicht gleichzeitig die Peptid-Carbonylgruppen reduziert, in einem inerten Lösungsmittel wie Methanol bei Temperaturen zwischen etwa -10 und +30°.

Eine Ketogruppe in einer Verbindung der Formel I kann auch durch reduktive Aminierung in eine CH(NH2)-Gruppe umgewandelt werden. Man kann ein- oder mehrstufig reduktiv aminieren. So kann man z. B. die Ketoverbindung mit Ammoniumsalzen, z. B. Ammoniumacetat, und NaCNBH3 behandeln, vorzugsweise in einem inerten Lösungsmittel, z. B. einem Alkohol wie Methanol bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfeisäure, Benzoesäure, Salicylsäure, 2- oder 3-Phenyipropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Konservierungs-, Stabilisierungs-und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-163237 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in $^\circ$C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Ein Gemisch von 844 mg 3S-Hydroxy-4S-[N-tert.-butoxycarbonyl-L-phenylalanyl-N(imi)-(2,4-dinitrophenyl)-L-histidyl-amino]-6-methyl-heptansulfonsäuremorpholid ["3S-Hydroxy-4S-(BOC-Phe-(imi-DNP-His)-amino)-6-methylheptan-sulfonsäuremorpholid; erhältlich durch Reduktion von 1-Brom-3S-CBZ-amino-5-methyl-hexan-2-on mit LiAl(O-tert.-C₄H₉)₃H zu 1-Brom-3S-CBZ-amino-5-methyl-hexan-2S-ol (daneben das 2R-Epimere, F. 88$^\circ$; chromatographische Trennung), Umsetzung mit 2,2-Dimethoxypropan/p-Toluolsulfonsäure in Dichlormethan zu 2,2-Dimethyl-3-CBZ-4S-isobutyl-5S-brommethyl-oxazolidin, Reaktion mit NaJ in Aceton zu 2,2-Dimethyl-3-CBZ-4S-isobutyl-5S-jodmethyl-oxazolidin (F. 57$^\circ$), Reaktion mit Methansulfonsäure-morpholid (F. 95$^\circ$)-Lithiumdiisopropylamid/HMPT/THF zu 2,2-Dimethyl-3-CBZ-4S-isobutyl-5S-(2-morpholinosulfonyl-ethyl)-oxazolidin, Hydrogenolyse an 5%ig. Pd/C in Methanol zu 3S-Hydroxy-4S-amino-6-methyl-heptan-sulfonsäure-morpholid und Kondensation mit BOC-Phe-(imi-DNP-His)-OH/DCCl/HOBt], 860 mg 2-Mercaptoethanol, 10 ml DMF und 10 ml Wasser wird unter Rühren bei 20$^\circ$ mit wässeriger Na₂CO₃-Lösung auf pH 8 eingestellt und 2 Std. bei 20$^\circ$ gerührt. Nach üblicher Aufarbeitung erhält man 3S-Hydroxy-4S-(N-tert.-butoxycarbonyl-L-phenylalanyl-L-histidyl-amino)-6-methyl-heptan-sulfonsäure-morpholid ("3S-Hydroxy-4S-BOC-Phe-His-amino-6-methyl-heptan-suifonsäure-morpholid"), F. 165$^\circ$.

Analog erhält man durch Spaltung der entsprechenden (imi-DNP-His)-Derivate:

1-Phenylthio-3S-hydroxy-4S-BOC-Phe-His-amino-6-methyl-heptan
1-Phenylthio-3S-hydroxy-4S-BOC-Phe-His-amino-5-cyclohexyl-pentan
1R-Isopropyl-1-phenylthio-3S-hydroxy-4S-BOC-Phe-His-amino-5-cyclohexyl-pentan

1S-Isopropyl-1-phenylthio-3S-hydroxy-4S-BOC-Phe-His-amino-5-cyclohexyl-pentan
1-Phenylsulfinyl-3S-hydroxy-4S-BOC-Phe-His-amino-6-methyl-heptan
1-Phenylsulfinyl-3S-hydroxy-4S-BOC-Phe-His-amino-5-cyclohexyl-pentan
1R-Isopropyl-1-phenylsulfinyl-3S-hydroxy-4S-BOC-Phe-His-amino-5-cyclohexyl-pentan
1S-Isopropyl-1-phenylsulfinyl-3S-hydroxy-4S-BOC-Phe-His-amino-5-cyclohexyl-pentan
3S-Hydroxy-4S-(2-Benzyl-4-oxo-5.5-dimethyl-hexanoyl-His-amino)-6-methyl-heptan-sulfonsäure-morpholid
3S-Hydroxy-4S-(2-Benzyl-4-oxo-5,5-dimethyl-hexanoyl-His-amino)-5-cyclohexyl-pentan-sulfonsäure-morpholid
3S-Hydroxy-4S-BOC-Ftr-Pro-Phe-His-amino-6-methyl-heptansulfonsäure-morpholid
3S-Hydroxy-4S-BOC-Ftr-Pro-Phe-His-amino-5-cyclohexylpentan-sulfonsäure-morpholid
1R-Isopropyl-3S-hydroxy-4S-BOC-Ftr-Pro-Phe-His-amino-6-methyl-heptan-sulfonsäure-morpholid
1S-Isopropyl-3S-hydroxy-4S-BOC-Ftr-Pro-Phe-His-amino-6-methyl-heptan-sulfonsäure-morpholid
1R-Isopropyl-3S-hydroxy-4S-BOC-Ftr-Pro-Phe-His-amino-5-cyclohexyl-pentan-sulfonsäure-morpholid
1S-Isopropyl-3S-hydroxy-4S-BOC-Ftr-Pro-Phe-His-amino-5-cyclohexyl-pentan-sulfonsäure-morpholid
3S-Hydroxy-4S-isovaleryl-Ftr-Pro-Phe-His-amino-6-methyl-heptan-sulfonsäure-morpholid
3S-Hydroxy-4S-isovaleryl-Ftr-Pro-Phe-His-amino-5-cyclohexyl-pentan--sulfonsäure-morpholid.

Beispiel 2

Man löst 1 g 3S-Hydroxy-4S-bis-(1-naphthylmethyl)-acetyl-(imi-BOM-His)-amino-6-methyl-heptan-sulfonsäure-morpholid [F. 110$^\circ$; erhältlich durch Reaktion von 3S-Hydroxy-4S-amino-6-methyl-heptan-sulfonsäure-morpholid mit BOC-(imi-BOM-His)-OH/DCCl/HOBt zu 3S-Hydroxy-4-BOC-(imi-BOM-His)-amino-6-methyl-heptan-sulfonsäure-morpholid (F. 56$^\circ$), Abspaltung der BOC-Gruppe mit 4n HCl in Dioxan und Acylierung mit Bis-(1-naphthylmethyl)-essigsäure] in 10 ml Methanol, hydriert an 0,5 g 10%ig. Pd-C bei 20$^\circ$ und 1 bar, filtriert, dampft ein und erhält 3S-Hydroxy-4S-bis-(1-naphthylmethyl)-acetyl-His-amino-6-methyl-heptan-sulfonsäure-morpholid, F. 130$^\circ$ (Zers.).

Analog sind durch Hydrogenolyse der entsprechenden BOM-Derivate erhältlich:

3S-Hydroxy-4S-bis-(1-naphthylmethyl)-acetyl-His-amino-5-cyclohexyl-pentan-sulfonsäure-morpholid, F. 133$^\circ$ [erhältlich über 1-Brom-3S-BOC-amino-4-cyclohexyl-butan-2-on (F. 86$^\circ$), 1-Brom-3S-BOC-amino-4-cyclohexyl-butan-2S-ol (Öl; daneben das 2R-Epimere), 2,2-Dimethyl-3-BOC-4S-cyclohexylmethyl-5S-brommethyl-oxazolidin (F. 102-103°), 2,2-Dimethyl-3-BOC-4S-cyclohexylmethyl-5S-jodmethyl-oxazolidin (F. 93°), 2,2-Dimethyl-3-BOC-4S-cyclohexylmethyl-5S-(2-morpholino-sulfonyl-ethyl)-oxazolidin, 3S-Hydroxy-4S-amino-5-cyclohexyl-pentan-sulfonsäure-morpholid, 3S-Hydroxy-4S-BOC-(imi-BOM-His)-amino-5-cyclohexyl-pentan-sulfonsäure-morpholid (F. 165°) und 3S-Hydroxy-4S-bis-(1-naphthylmethyl)-acetyl-(imi-BOM-His)-amino-5-cyclohexyl-pentan-sulfonsäure-morpholid (F. 114-115°)];

3S-Hydroxy-4S-BOC-Phe-His-amino-5-cyclohexyl-pentan-sulfonsäure-morpholid, F. 132° [erhältlich aus 3S-Hydroxy-4S-BOC-Phe-(imi-BOM-His)-amino-5-cyclohexyl-pentan-sulfonsäure-morpholid, F. 94°];

1R-Isopropyl-3S-hydroxy-4S-BOC-Phe-His-amino-6-methyl-heptan-sulfonsäure-morpholid,

1S-Isopropyl-3S-hydroxy-4S-BOC-Phe-His-amino-6-methyl-heptan-sulfonsäure-morpholid,

1R-Isopropyl-3S-hydroxy-4S-BOC-Phe-His-amino-5-cyclohexyl-pentan-sulfonsäure-morpholid, F. 116-117° [erhältlich über 1-Brom-3S-CBZ-amino-4-cyclohexyl-butan-2-on (F. 66°), 1-Brom-3S-CBZ-amino-4-cyclohexyl-butan-2S-ol (Öl; daneben das 2R-Epimere, F. 125°), 2,2-Dimethyl-3-CBZ-4S-cyclohexylmethyl-5S-brommethyl-oxazolidin (Öl), 2,2-Dimethyl-3-CBZ-4S-cyclohexylmethyl-5S-jodmethyl-oxazolidin (F. 67°), Reaktion desselben mit 2-Methyl-propan-sulfonsäure-morpholid (F. 82°)/Lithiumdiisopropylamid/HMPT zu 2,2-Dimethyl-3-CBZ-4S-cyclohexylmethyl-5S-(2R-isopropyl-2-morpholinosulfonyl-ethyl)-oxazolidin (Öl; daneben das 2S-Isopropyl-Epimere "A", F. 84°), 1R-Isopropyl-3S-hydroxy-4S-amino-5-cyclohexyl-pentan-sulfonsäure-morpholid und dessen Reaktion mit BOC-Phe-(imi-BOM-His)-OH (F. 182°) zu 1R-Isopropyl-3S-hydroxy-4S-BOC-Phe-(imi-Bom-His)-amino-5-cyclohexyl-pentan-sulfonsäure-morpholid (F. 118-120°)]; 1S-Isopropyl-3S-hydroxy-4S-BOC-Phe-His-amino-5-cyclohexyl-pentan-sulfonsäure-morpholid, F. 114-116° [erhältlich über "A", 1S-Isopropyl-3S-hydroxy-4S-amino-5-cyclohexyl-pentan-sulfonsäure-morpholid und 1S-Isopropyl-3S-hydroxy-4S-BOC-Phe-(imi-Bom-His)-amino-5-cyclohexyl-pentan-sulfonsäure-morpholid (F. 108-109°)];

3S-Hydroxy-4S-BOC-Phe-His-amino-6-methyl-heptan-sulfonsäure-pyrrolidid
3S-Hydroxy-4S-BOC-Phe-His-amino-5-cyclohexyl-pentan-sulfonsäure-pyrrolidid
1R-Isopropyl-3S-hydroxy-4S-BOC-Phe-His-amino-6-methyl-heptan-sulfonsäure-pyrrolidid
1S-Isopropyl-3S-hydroxy-4S-BOC-Phe-His-amino-6-methyl-heptan-sulfonsäure-pyrrolidid
1R-Isopropyl-3S-hydroxy-4S-BOC-Phe-His-amino-5-cyclohexyl-pentan-sulfonsäure-pyrrolidid
1S-Isopropyl-3S-hydroxy-4S-BOC-Phe-His-amino-5-cyclohexyl-pentan-sulfonsäure-pyrrolidid

3S-Hydroxy-4S-BOC-Phe-His-amino-6-methyl-heptan-sulfonsäure-piperidid
3S-Hydroxy-4S-BOC-Phe-His-amino-5-cyclohexyl-pentan-sulfonsäure-piperidid
1R-Isopropyl-3S-hydroxy-4S-BOC-Phe-His-amino-6-methyl-heptan-sulfonsäure-piperidid
1S-Isopropyl-3S-hydroxy-4S-BOC-Phe-His-amino-6-methyl-heptan-sulfonsäure-piperidid
1R-Isopropyl-3S-hydroxy-4S-BOC-Phe-His-amino-5-cyclohexyl-pentan-sulfonsäure-piperidid
1S-Isopropyl-3S-hydroxy-4S-BOC-Phe-His-amino-5-cyclohexyl-pentan-sulfonsäure-piperidid

3S-Hydroxy-4S-BOC-Phe-His-amino-6-methyl-heptan-sulfonsäure-piperazid
3S-Hydroxy-4S-BOC-Phe-His-amino-5-cyclohexyl-pentan-sulfonsäure-piperazid
1R-Isopropyl-3S-hydroxy-4S-BOC-Phe-His-amino-6-methyl-heptan-sulfonsäure-piperazid
1S-Isopropyl-3S-hydroxy-4S-BOC-Phe-His-amino-6-methylheptan-sulfonsäure-piperazid
1R-Isopropyl-3S-hydroxy-4S-BOC-Phe-His-amino-5-cyclohexyl-pentan-sulfonsäure-piperazid
1S-Isopropyl-3S-hydroxy-4S-BOC-Phe-His-amino-5-cyclohexyl-pentan-sulfonsäure-piperazid
3S-Hydroxy-4S-BOC-Phe-His-amino-6-methyl-heptan-sulfonsäure-dimethylamid
3S-Hydroxy-4S-BOC-Phe-His-amino-5-cyclohexyl-pentan-sulfonsäure-dimethylamid
1R-Isopropyl-3S-hydroxy-4S-BOC-Phe-His-amino-6-methyl-heptan-sulfonsäure-dimethylamid
1S-Isopropyl-3S-hydroxy-4S-BOC-Phe-His-amino-6-methyl-heptan-sulfonsäure-dimethylamid
1R-Isopropyl-3S-hydroxy-4S-BOC-Phe-His-amino-5-cyclohexyl-pentan-sulfonsäure-dimethylamid
1S-Isopropyl-3S-hydroxy-4S-BOC-Phe-His-amino-5-cyclohexyl-pentan-sulfonsäure-dimethylamid

3S-Hydroxy-4S-BOC-Phe-His-amino-6-methyl-heptan-sulfonsäure-(N-benzyl-N-butyl-amid)
3S-Hydroxy-4S-BOC-Phe-His-amino-5-cyclohexyl-pentan-sulfonsäure-(N-benzyl-N-butyl-amid)
1R-Isopropyl-3S-hydroxy-4S-BOC-Phe-His-amino-6-methyl-heptan-sulfonsäure-(N-benzyl-N-butyl-amid)
1S-Isopropyl-3S-hydroxy-4S-BOC-Phe-His-amino-6-methyl-heptan-sulfonsäure-(N-benzyl-N-butyl-amid)
1R-Isopropyl-3S-hydroxy-4S-BOC-Phe-His-amino-5-cyclohexyl-pentan-sulfonsäure-(N-benzyl-N-butyl-amid)
1S-Isopropyl-3S-hydroxy-4S-BOC-Phe-His-amino-
5-cyclohexyl-pentan-sulfonsäure-(N-benzyl-N-butyl-
amid)

3S-Hydroxy-4S-BOC-Phe-His-amino-6-methyl-
heptan-sulfonsäure-(N-butyl-amid)
3S-Hydroxy-4S-BOC-Phe-His-amino-5-cyclohexyl-
pentan-sulfonsäure-(N-butyl-amid)
1R-Isopropyl-3S-hydroxy-4S-BOC-Phe-His-amino-
6-methyl-heptan-sulfonsäure-(N-butyl-amid)
1S-Isopropyl-3S-hydroxy-4S-BOC-Phe-His-amino-
6-methyl-heptan-sulfonsäure-(N-butyl-amid)
1R-Isopropyl-3S-hydroxy-4S-BOC-Phe-His-amino-
5-cyclohexyl-pentan-sulfonsäure-(N-butyl-amid)
1S-Isopropyl-3S-hydroxy-4S-BOC-Phe-His-amino-
5-cyclohexyl-pentan-sulfonsäure-(N-butyl-amid)

3S-Hydroxy-4S-BOC-Phe-His-amino-6-methyl-
heptan-sulfonsäure-(N-isopentylamid)
3S-Hydroxy-4S-BOC-Phe-His-amino-5-cyclohexyl-
pentan-sulfonsäure-(N-isopentylamid)
1R-Isopropyl-3S-hydroxy-4S-BOC-Phe-His-amino-
6-methyl-heptan-sulfonsäure-(N-isopentylamid)
1S-Isopropyl-3S-hydroxy-4S-BOC-Phe-His-amino-
6-methyl-heptan-sulfonsäure-(N-isopentylamid)
1R-Isopropyl-3S-hydroxy-4S-BOC-Phe-His-amino-
5-cyclohexyl-pentan-sulfonsäure-(N-isopentylamid)
1S-Isopropyl-3S-hydroxy-4S-BOC-Phe-His-amino-
5-cyclohexyl-pentan-sulfonsäure-(N-isopentylamid)

1-Phenylsulfonyl-3S-hydroxy-4S-BOC-Phe-His-
amino-6-methyl-heptan
1-Phenylsulfonyl-3S-hydroxy-4S-BOC-Phe-His-
amino-5-cyclohexyl-pentan
1R-Isopropyl-1-phenylsulfonyl-3S-hydroxy-4S-BOC-
Phe-His-amino-6-methyl-heptan
1S-Isopropyl-1-phenylsulfonyl-3S-hydroxy-4S-BOC-
Phe-His-amino-6-methyl-heptan
1R-Isopropyl-1-phenylsulfonyl-3S-hydroxy-4S-BOC-
Phe-His-amino-5-cyclohexyl-pentan
1S-Isopropyl-1-phenylsulfonyl-3S-hydroxy-4S-BOC-
Phe-His-amino-5-cyclohexyl-pentan.

Analog erhält man die folgenden 6-Methyl-
heptan-sulfonsäure-morpholide:

3S-Hydroxy-4S-BOC-Bia-His-amino-
3S-Hydroxy-4S-BOC-Cal-His-amino-
3S-Hydroxy-4S-BOC-Hph-His-amino-
3S-Hydroxy-4S-BOC-αNal-His-amino-
3S-Hydroxy-4S-BOC-Nle-His-amino-
3S-Hydroxy-4S-BOC-Trp-His-amino-
3S-Hydroxy-4S-BOC-Tyr-His-amino-
3S-Hydroxy-4S-BOC-Mal-His-amino-

3S-Hydroxy-4S-(2-benzyl-heptanoyl-His-amino)-
3S-Hydroxy-4S-(2-benzyl-3-phenyl-propionyl-His-amino)-
3S-Hydroxy-4S-(2-benzyl-4-phenyl-butyryl-His-
amino)-
3S-Hydroxy-4S-POA-His-amino-
3S-Hydroxy-4S-[2-(1-naphthylmethyl)-3-N-
isopropylcarbamoyl-propionyl-His-amino]-
3S-Hydroxy-4S-(2-benzyl-3-MC-propionyl-His-
amino)-
3S-Hydroxy-4S-(N-benzyl-N-butyl-carbamoyl-His-
amino)-
3S-Hydroxy-4S-BOC-Phe-(N-methyl-His)-amino-
3S-Hydroxy-4S-BOC-Pro-Phe-His-amino-
3S-Hydroxy-4S-BOC-Pro-Phe-(N-methyl-His)-
amino-
3S-Hydroxy-4S-BOC-His-Pro-Phe-His-amino-  (aus
dem Bis-imi-BOM-His-Derivat)
1R-Isopropyl-3S-hydroxy-4S-BOC-Phe-(N-methyl-
His)-amino-
1S-Isopropyl-3S-hydroxy-4S-BOC-Phe-(N-methyl-
His)-amino-.

Analog erhält man die folgenden 5-Cyclohexyl-
pentan-sulfonsäure-morpholide:

3S-Hydroxy-4S-BOC-Bia-His-amino-
3S-Hydroxy-4S-BOC-Cal-His-amino-
3S-Hydroxy-4S-BOC-Hph-His-amino-
3S-Hydroxy-4S-BOC-αNal-His-amino-
3S-Hydroxy-4S-BOC-Nle-His-amino-
3S-Hydroxy-4S-BOC-Trp-His-amino-
3S-Hydroxy-4S-BOC-Tyr-His-amino-
3S-Hydroxy-4S-BOC-Mal-His-amino-
3S-Hydroxy-4S-(2-benzyl-heptanoyl-His-amino)-
3S-Hydroxy-4S-(2-benzyl-3-phenyl-propionyl-His-
amino)-
3S-Hydroxy-4S-(2-benzyl-4-phenyl-butyryl-His-
amino)-
3S-Hydroxy-4S-POA-His-amino-
3S-Hydroxy-4S-[2-(1-naphthylmethyl)-3-N-
isopropylcarbamoyl-propionyl-His-amino]-
3S-Hydroxy-4S-(2-benzyl-3-MC-propionyl-His-
amino)-
3S-Hydroxy-4S-(N-benzyl-N-butyl-carbamoyl-His-
amino)-
3S-Hydroxy-4S-BOC-Phe-(N-methyl-His)-amino-
3S-Hydroxy-4S-BOC-Pro-Phe-His-amino-
3S-Hydroxy-4S-BOC-Pro-Phe-(N-methyl-His)-
amino-
3S-Hydroxy-4S-BOC-His-Pro-Phe-His-amino-  (aus
dem Bis-imi-BOM-His-Derivat)
1R-Isopropyl-3S-hydroxy-4S-BOC-Phe-(N-methyl-
His)-amino-
1S-Isopropyl-3S-hydroxy-4S-BOC-Phe-(N-methyl-
His)-amino-.

## Beispiel 3

Analog Beispiel 2 erhält man durch Hydrogenolyse von 3S-Hydroxy-4S-[5-CBZ-amino-hexanoyl-Phe-(imi-BOM-His)-amino]-5-cyclohexyl-pentan-sulfonsäure-morpholid [erhältlich durch Abspaltung der BOC-Gruppe aus 3S-Hydroxy-4S-BOC-Phe-(imi-BOM-His-amino)-5-cyclohexyl-pentan-sulfonsäure-morpholid und Reaktion mit 6-CBZ-amino-hexansäure] das 3S-Hydroxy-4S-(6-amino-hexanoyl-Phe-His-amino)-5-cyclohexyl-pentan-sulfonsäure-morpholid.

## Beispiel 4

Eine Lösung von 100 mg 3S-Hydroxy-4S-[4-trimethylammonium-butyryl-Phe-(imi-BOM-His)-amino]-5-cyclohexyl-pentan-sulfonsäure-morpholid-chlorid in 10 ml Methanol wird mit 100 mg 10%ig Pd-C und 500 mg Ammoniumformiat versetzt und 8 Std. bei 20° gerührt. Man filtriert, dampft ein, chromatographiert den Rückstand an Kieselgel mit Dichlorme than/Methanol und erhält 3S-Hydroxy-4S-(4-trimethylammonium-butyryl-Phe-His-amino)-5-cyclohexyl-pentan-sulfonsäure-morpholid-formiat.

Analog erhält man die folgenden 5-Cyclohexyl-pentan-sulfonsäure-morpholide:

3S-Hydroxy-4S-(isovaleryl-Phe-His-amino)-
3S-Hydroxy-4S-(3,3-dimethylbutyryl-Phe-His-amino)-
3S-Hydroxy-4S-(morpholinoacetyl-Phe-His-amino)-
3S-Hydroxy-4S-(PyOC-Phe-His-amino)-
3S-Hydroxy-4S-(IPOC-Phe-His-amino)-
3S-Hydroxy-4S-(MC-Phe-His-amino)-
3S-Hydroxy-4S-[4-(4-pyridyl)-butyryl-Phe-His-amino)-
3S-Hydroxy-4S-(6-amino-hexanoyl-Phe-His-amino)-
3S-Hydroxy-4S-(4-dimethylaminobutyryl-Phe-His-amino)-
3S-Hydroxy-4S-(6-dimethylaminohexanoyl-Phe-His-amino)-
3S-Hydroxy-4S-(6-trimethylammoniumhexanoyl-Phe-His-amino)-, Formiat.

Analog erhält man die folgenden 6-Methyl-heptan-sulfonsäure-morpholide:

3S-Hydroxy-4S-(4-trimethylammonium-butyryl-Phe-His-amino-Formiat
3S-Hydroxy-4S-(isovaleryl-Phe-His-amino)-
3S-Hydroxy-4S-(3,3-dimethylbutyryl-Phe-His-amino)-
3S-Hydroxy-4S-(morpholinoacetyl-Phe-His-amino)-
3S-Hydroxy-4S-(PyOC-Phe-His-amino)-
3S-Hydroxy-4S-(IPOC-Phe-His-amino)-
3S-Hydroxy-4S-(MC-Phe-His-amino)-

3S-Hydroxy-4S-[4-(4-pyridyl)-butyryl-Phe-His-amino)-
3S-Hydroxy-4S-(6-amino-hexanoyl-Phe-His-amino)-
3S-Hydroxy-4S-(4-dimethylaminobutyryl-Phe-His-amino)-
3S-Hydroxy-4S-(6-dimethylaminohexanoyl-Phe-His-amino)-
3S-Hydroxy-4S-(6-trimethylammoniumhexanoyl-Phe-His-amino)-, Formiat.

## Beispiel 5

Eine Lösung von 3,51 g 3S-Hydroxy-4S-(H-Gly-amino)-6-methyl-heptan-sulfonsäure-morpholid (erhältlich durch Kondensation von BOC-Gly-OH mit 3S-Hydroxy-4S-amino-6-methyl-heptan-sulfonsäure-morpholid und anschließende Abspaltung der BOC-Gruppe mit 4n HCl) in 60 ml Dichlormethan wird unter Rühren mit 2,65 g BOC-Phe-OH, 1,25 g HOBt und einer Lösung von 2,06 g DCCI in 50 ml Dichlormethan versetzt. Man rührt 14 Std. bei 2-6°, filtriert den ausgefallenen Dicyclohexylharnstoff ab, dampft das Filtrat ein, arbeitet wie üblich auf und erhält 3S-Hydroxy-4S-BOC-Phe-Gly-amino-6-methyl-heptan-sulfonsäure-morpholid.

## Beispiel 6

Analog Beispiel 5 erhält man aus BOC-Phe-Abu-OH und 3S-Hydroxy-4S-amino-5-cyclohexyl-pentan-sulfonsäure-morpholid das 3S-Hydroxy-4S-BOC-Phe-Abu-amino-5-cyclohexylpentan-sulfonsäure-morpholid.

Analog erhält man die folgenden 6-Methyl-heptan-sulfonsäure morpholide:

3S-Hydroxy-4S-BOC-Phe-Abu-amino-
3S-Hydroxy-4S-BOC-Phe-Ala-amino-
3S-Hydroxy-4S-BOC-Phe-βAla-amino-
3S-Hydroxy-4S-BOC-Phe-Asn-amino-
3S-Hydroxy-4S-BOC-Phe-Gln-amino-
3S-Hydroxy-4S-BOC-Phe-Gly-amino-
3S-Hydroxy-4S-BOC-Phe-Leu-amino-
3S-Hydroxy-4S-BOC-Phe-Met-amino-
3S-Hydroxy-4S-BOC-Phe-Nle-amino-
3S-Hydroxy-4S-BOC-Phe-(3-Pyr)-amino-;

die folgenden 5-Cyclohexyl-pentan-sulfonsäure-morpholide:

3S-Hydroxy-4S-BOC-Phe-Ala-amino-
3S-Hydroxy-4S-BOC-Phe-βAla-amino-
3S-Hydroxy-4S-BOC-Phe-Asn-amino-
3S-Hydroxy-4S-BOC-Phe-Gln-amino-
3S-Hydroxy-4S-BOC-Phe-Gly-amino-
3S-Hydroxy-4S-BOC-Phe-Leu-amino-

3S-Hydroxy-4S-BOC-Phe-Met-amino-
3S-Hydroxy-4S-BOC-Phe-Nle-amino-
3S-Hydroxy-4S-BOC-Phe-(3-Pyr)-amino-;

die folgenden 1R-Isopropyl- und 1S-Isopropyl-6-methyl-heptan-sulfonsäure-morpholide:

-3S-hydroxy-4S-BOC-Phe-Abu-amino-
-3S-hydroxy-4S-BOC-Phe-Ala-amino-
-3S-hydroxy-4S-BOC-Phe-$\beta$Ala-amino-
-3S-hydroxy-4S-BOC-Phe-Asn-amino-
-3S-hydroxy-4S-BOC-Phe-Gln-amino-
-3S-hydroxy-4S-BOC-Phe-Gly-amino-
-3S-hydroxy-4S-BOC-Phe-Leu-amino-
-3S-hydroxy-4S-BOC-Phe-Met-amino-
-3S-hydroxy-4S-BOC-Phe-Nle-amino-
-3S-hydroxy-4S-BOC-Phe-(3-Pyr)-amino-;

sowie die folgenden 1R-Isopropyl- und 1S-Isopropyl-5-cyclohexyl-pentan-sulfonsäure-morpholide:

-3S-hydroxy-4S-BOC-Phe-Abu-amino-
-3S-hydroxy-4S-BOC-Phe-Ala-amino-
-3S-hydroxy-4S-BOC-Phe-$\beta$Ala-amino-
-3S-hydroxy-4S-BOC-Phe-Asn-amino-
-3S-hydroxy-4S-BOC-Phe-Gln-amino-
-3S-hydroxy-4S-BOC-Phe-Gly-amino-
-3S-hydroxy-4S-BOC-Phe-Leu-amino-
-3S-hydroxy-4S-BOC-Phe-Met-amino-
-3S-hydroxy-4S-BOC-Phe-Nle-amino-
-3S-hydroxy-4S-BOC-Phe-(3-Pyr)-amino-.

Beispiel 7

Eine Lösung von 677 mg 1-Phenylthio-3S-hydroxy-4S-BOC-Phe-His-amino-5-cyclohexylpentan und 190 mg 3-Chlorperbenzoesäure in 8 ml Chloroform wird 16 Std. bei 0° stehengelassen. Nach üblicher Aufarbeitung erhält man 1-Phenylsulfinyl-3S-hydroxy-4S-BOC-Phe-His-amino-5-cyclohexyl-pentan.

Beispiel 8

Analog Beispiel 7, aber mit 440 mg 3-Chlorperbenzoesäure erhält man 1-Phenylsulfonyl-3S-hydroxy-4S-BOC-Phe-His-amino-5-cyclohexyl-pentan.

Beispiel 9

Eine Lösung von 1 g 3S-Hydroxy-4S-BOC-Phe-His-amino-6-methyl-heptan-sulfonsäure-morpholid in 20 ml 4 n HCl in Dioxan wird 30 Min.

bei 20° gerührt und dann eingedampft. Man erhält 3S-Hydroxy-4S-H-Phe-His-amino-6-methyl-heptan-sulfonsäure-morpholid.

Analog erhält man aus den entsprechenden BOC-Derivaten:

3S-Hydroxy-4S-H-Phe-His-amino-5-cyclohexyl-pentan-sulfonsäure-morpholid
1R-Isopropyl-3S-hydroxy-4S-H-Phe-His-amino-6-methyl-heptan-sulfonsäure-morpholid
1S-Isopropyl-3S-hydroxy-4S-H-Phe-His-amino-6-methyl-heptan-sulfonsäure-morpholid.

Beispiel 10

Man löst 1 g 3S-Hydroxy-4S-CBZ-Phe-Gly-amino-6-methyl-heptan-sulfonsäure-morpholid in 10 ml Ethanol, hydriert an 0,5 g 10%igem Pd-C bei 20° und 1 bar bis zum Ende der $H_2$-Aufnahme, filtriert, dampft ein und erhält nach chromatographischer Reinigung 3S-Hydroxy-4S-H-Phe-Gly-amino-6-methyl-heptan-sulfonsäure-morpholid.

Beispiel 11

a) Analog Beispiel 5 erhält man aus BOC-Phe-Gly-OH und 3-Oxo-4S-amino-5-cyclohexylpentanoyl-sulfonsäure-morpholid (erhältlich durch Oxydation von 3S-Hydroxy-4S-BOC-amino-5-cyclohexyl-pentan-sulfonsäure-morpholid mit $CrO_3$/Pyridin und Abspaltung der BOC-Gruppe) das 3-Oxo-4S-(BOC-Phe-Gly-amino)-5-cyclohexylpentan-sulfonsäure-morpholid.

b) Eine Lösung von 1 g des vorstehenden Amids in 25 ml $CH_3OH$ wird an 0,1 g 10 %ig. Pd-C bei 20° und 1 bar bis zum Stillstand hydriert. Nach Filtrieren und Eindampfen erhält man ein Gemisch von 3R- und 3S-Hydroxy-4S-(BOC-Phe-Gly-amino)-5-cyclohexylpentan-sulfonsäure-morpholid, das chromatographisch getrennt werden kann.

Beispiel 12

Eine Lösung von 636 mg 3-Oxo-4S-(BOC-Phe-Gly-amino)-5-cyclohexylpentan-sulfonsäure-morpholid und 1,43 g $Na_2CO_3$. 10 $H_2O$ in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert. Man filtriert, dampft das Filtrat ein und erhält nach üblicher Aufarbeitung ein Gemisch von 3R- und 3S-Amino-4S-(BOC-Phe-Gly-amino)-5-cyclohexyl-pentansulfonsäure-morpholid, das chromatographisch getrennt werden kann.

Beispiel 13

Analog Beispiel 1 erhält man durch Spaltung der entsprechenden imi-DNP-His-Derivate die folgenden 3S-Hydroxy-5-cyclohexyl-pentansulfonsäure-morpholide:

2S-Isopropyl-4S-(2R,S-benzyl-4-oxo-5,5-dimethylhexanoyl-His-amino)-, F. 123-124°
2S-Isopropyl-4S-[2-(1-napthylmethyl)-3-MC-propionyl-His-amino]-, Methansulfonat, 2 Epimere, F. 160-161° (Zers.) bzw. 176-177° (Zers.)
2S-Isopropyl-4S-bis-(1-naphthylmethyl)-acetyl-His-amino-, Methansulfonat, F. 166° (Zers.)
2S-Isopropyl-4S-(2-benzyl-3-tert.-butyl-sulfonyl-propionyl-His-amino)-, 2 Epimere, F. 103° (Methansulfonat, F. 81°) bzw. 110° (Methansulfonat, F. 83°)
4S-(2-Benzyl-3-tert.-butyl-sulfonyl-propionyl-His-amino)-, 2 Epimere, F. 120° (Hydrochlorid, F. 113-114°) bzw. 113° (Hydrochlorid, F. 116-117°)
4S-[2-(1-Naphthylmethyl)-3-MC-propionyl-His-amino]-, 2 Epimere, F. 120° (Hydrochlorid, F. 126-127°) bzw. 130° (Hydrochlorid, F. 101-102°.)
1R,S-Methyl-4S-(2-benzyl-3-tert.-butylsulfonyl-propionyl-His-amino)-, 2 Epimere, F. 104° (Hydrochlorid, F. 135°) bzw. 123° (Hydrochlorid, F. 150°)
4-[2-(1-Naphthylmethyl)-3-(N-3-dimethylaminopropyl-carbamoyl)-propionyl-His-amino]-, Dihydrochlorid, 2 Epimere, F. 127-129° bzw. 133-136°
1R,S-Methyl-4S-[2-(1-naphthylmethyl)-3-(4-BOC-aminopiperidinocarbonyl)-propionyl-His-amino]-
4S-BOC-Mal-His-amino-, F. 153° (Zers.)
4S-(4-BOC-amino-piperidinocarbonyl-Phe-His-amino)-
4S-(4-BOC-amino-piperidinocarbonyl-Mal-His-amino)-
1R,S-Methyl-4S-(4-BOC-amino-piperidinocarbonyl-Phe-His-amino)-
1R,S-Methyl-4S-(4-BOC-amino-piperidinocarbonyl-Mal-His-amino)-
1R,S-Isopropyl-4S-(4-BOC-amino-piperidinocarbonyl-Phe-His-amino)-
1R,S-Isopropyl-4S-(4-BOC-amino-piperidinocarbonyl-Mal-His-amino)-
4S-(2-BOC-amino-2-methyl-propionyl-Phe-His-amino)-
4S-(2-BOC-amino-2-methyl-propionyl-Mal-His-amino)-
1R,S-Methyl-4S-(2-BOC-amino-2-methyl-propionyl-Phe-His-amino)-
1R,S-Methyl-4S-(2-BOC-amino-2-methyl-propionyl-Mal-His-amino)-
1R,S-Isopropyl-4S-(2-BOC-amino-2-methyl-propionyl-Phe-His-amino)-
1R,S-Isopropyl-4S-(2-BOC-amino-2-methyl-propionyl-Mal-His-amino)-
4S-(3-BOC-amino-3-methyl-butyryl-Phe-His-amino)-
4S-(3-BOC-amino-3-methyl-butyryl-Mal-His-amino)-
1R,S-Methyl-4S-(3-BOC-amino-3-methyl-butyryl-Phe-His-amino)-
1R,S-Methyl-4S-(3-BOC-amino-3-methyl-butyryl-Mal-His-amino)-
1R,S-Isopropyl-4S-(3-BOC-amino-3-methyl-butyryl-Phe-His-amino)-
1R,S-Isopropyl-4S-(3-BOC-amino-3-methyl-butyryl-Mal-His-amino)-
4S-(8-BOC-amino-octanoyl-Phe-His-amino)-
4S-(8-BOC-amino-octanoyl-Mal-His-amino)-
1R,S-Methyl-4S-(8-BOC-amino-octanoyl-Phe-His-amino)-
1R,S-Methyl-4S-(8-BOC-amino-octanoyl-Mal-His-amino)-
1R,S-Isopropyl-4S-(8-BOC-amino-octanoyl-Phe-His-amino)-
1R,S-Isopropyl-4S-(8-BOC-amino-octanoyl-Mal-His-amino)-
4S-[4-BOC-amino-piperidinocarbonyl-Phe-(2-Tia)-amino]-
4S-[4-BOC-amino-piperidinocarbonyl-Mal-(2-Tia)-amino]-
1R,S-Methyl-4S-[4-BOC-amino-piperidinocarbonyl-Phe-(2-Tia)-amino]-
1R,S-Methyl-4S-[4-BOC-amino-piperidinocarbonyl-Mal-(2-Tia)-amino]-
1R,S-Isopropyl-4S-[4-BOC-amino-piperidinocarbonyl-Phe-(2-Tia)-amino]-
1R,S-Isopropyl-4S-[4-BOC-amino-piperidinocarbonyl-Mal-(2-Tia)-amino]-

sowie die folgenden 3S-Hydroxy-5-cyclohexyl-pentansulfonsäure-N-butylamide:

4S-(2-Benzyl-3-tert.-butylsulfonyl-propionyl-His-amino)-
1R,S-Methyl-4S-(2-benzyl-3-tert.-butylsulfonyl-propionyl-His-amino)-
1R,S-Isopropyl-4S-(2-benzyl-3-tert.-butylsulfonyl-propionyl-His-amino)-
4S-(2-Benzyl-3-MC-propionyl-His-amino)-
1R,S-Methyl-4S-(2-benzyl-3MC-propionyl-His-amino)-
1R,S-Isopropyl-4S-(2-benzyl-3MC-propionyl-His-amino)-
4S-(3-BOC-amino-3-methyl-butyryl-Mal-His-amino)-
1R,S-Methyl-4S-(3-BOC-amino-3-methyl-butyryl-Mal-His-amino)-
1R,S-Isopropyl-4S-(3-BOC-amino-3-methyl-butyryl-Mal-His-amino)-
4S-(4-BOC-amino-piperidinocarbonyl-Mal-His-amino)-
1R,S-Methyl-4S-(4-BOC-amino-piperidinocarbonyl-Mal-His-amino)-
1R,S-Isopropyl-4S-(4-BOC-amino-piperidinocarbonyl-Mal-His-amino)-

Beispiel 14

Analog Beispiel 9 erhält man aus den entsprechenden BOC-Derivaten mit 4n HCl in Dioxan die folgenden 3S-Hydroxy-5-cyclohexyl-pentansulfonsäure-morpholide:

1R,S-Methyl-4S-[2-(1-naphthylmethyl)-3-(4-aminopiperidinocarbonyl)-propionyl-His-amino]-, Dihydrochlorid, 2 Epimere, F. 158° bzw. 167°

4S-(4-Amino-piperidinocarbonyl-Phe-His-amino)-
4S-(4-Amino-piperidinocarbonyl-Mal-His-amino)-
1R,S-Methyl-4S-(4-amino-piperidinocarbonyl-Phe-His-amino)-
1R,S-Methyl-4S-(4-amino-piperidinocarbonyl-Mal-His-amino)-
1R,S-Isopropyl-4S-(4-amino-piperidinocarbonyl-Phe-His-amino)-
1R,S-Isopropyl-4S-(4-amino-piperidinocarbonyl-Mal-His-amino)-
4S-(2-Amino-2-methyl-propionyl-Phe-His-amino)-
4S-(2-Amino-2-methyl-propionyl-Mal-His-amino)-
1R,S-Methyl-4S-(2-amino-2-methyl-propionyl-Phe-His-amino)-
1R,S-Methyl-4S-(2-amino-2-methyl-propionyl-Mal-His-amino)-
1R,S-Isopropyl-4S-(2-amino-2-methyl-propionyl-Phe-His-amino)-
1R,S-Isopropyl-4S-(2-amino-2-methyl-propionyl-Mal-His-amino)-
4S-(3-Amino-4-methyl-butyryl-Phe-His-amino)-
4S-(3-Amino-4-methyl-butyryl-Mal-His-amino)-
1R,S-Methyl-4S-(3-amino-3-methyl-butyryl-Phe-His-amino)-
1R,S-Methyl-4S-(3-amino-3-methyl-butyryl-Mal-His-amino)-
1R,S-Isopropyl-4S-(3-amino-3-methyl-butyryl-Phe-His-amino)-
1R,S-Isopropyl-4S-(3-amino-3-methyl-butyryl-Mal-His-amino)-
4S-(8-Aminooctanoyl-Phe-His-amino)-
4S-(8-Aminooctanoyl-Mal-His-amino)-
1R,S-Methyl-4S-(8-aminooctanoyl-Phe-His-amino)-
1R,S-Methyl-4S-(8-aminooctanoyl-Mal-His-amino)-
1R,S-Isopropyl-4S-(8-aminooctanoyl-Phe-His-amino)-
1R,S-Isopropyl-4S-(8-aminooctanoyl-Mal-His-amino)-

4S-[4-Amino-piperidinocarbonyl-Phe-(2-Tia)-amino]-
4S-[4-Amino-piperidinocarbonyl-Mal-(2-Tia)-amino]-
1R,S-Methyl-4S-[4-amino-piperidinocarbonyl-Phe-(2-Tia)-amino]-
1R,S-Methyl-4S-[4-amino-piperidinocarbonyl-Mal-(2-Tia)-amino]-
1R,S-Isopropyl-4S-[4-amino-piperidinocarbonyl-Phe-(2-Tia)-amino]-
1R,S-Isopropyl-4S-[4-amino-piperidinocarbonyl-Mal-(2-Tia)-amino]-

sowie die folgenden 3S-Hydroxy-5-cyclohexyl-pentansulfonsäure-N-butylamide:

4S-(3-Amino-3-methyl-butyryl-Mal-His-amino)-
1R,S-Methyl-4S-(3-amino-3-methyl-butyryl-Mal-His-amino)-
1R,S-Isopropyl-4S-(3-amino-3-methyl-butyryl-Mal-His-amino)-
4S-(4-Amino-piperidinocarbonyl-Mal-His-amino)-
1R,S-Methyl-4S-(4-amino-piperidinocarbonyl-Mal-His-amino)-
1R,S-Isopropyl-4S-(4-amino-piperidinocarbonyl-Mal-His-amino)-.

Die nachstehenden Beispiele betreffen pharmazeutische Zubereitungen.

Beispiel A: Tabletten

Ein Gemisch von 1 kg 1R,S-Methyl-3S-hydroxy-4S-[2-(1-naphthylmethyl)-3-(4-aminopiperidinocarbonyl)-propionyl-His-amino]-5-cyclohexyl-pentansulfonsäure-morpholid-dihydrochlorid (F. 167°), 4 kg Lactose, 1,2 kg Kartoffelstärke, 200 g Talk und 100 g Magnesiumstearat wird in üblicher Weise granuliert und dann zu Tabletten verpreßt, derart, daß jede Tablette 50 mg Wirkstoff enthält.

Beispiel B: Dragees

Analog Beispiel A werden Tabletten gepreßt, die anschliessend in üblicher Weise mit einem Überzug aus Saccharose, Kartoffelstärke, Talk, Tragant, Farbstoff und Vanille-Aroma überzogen werden.

Beispiel C: Kapseln

Man füllt 1 kg 1R,S-Methyl-3S-hydroxy-4S-(2-benzyl-3-tert.-butyl-sulfonyl-propionyl-His-amino)-5-cyclohexyl-pentan-sulfonsäure-morpholid-hydrochlorid (F. 150 °) in üblicher Weise in Hartgelatinekapseln, so daß jede Kapsel 100 mg Wirkstoff enthält.

Beispiel D: Injektionsgläser

Eine Lösung von 100 g 3S-Hydroxy-4S-BOC-Phe-His-amino-5-cyclohexyl-pentan-sulfonsäure-morpholid und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.

Beispiel E: Suppositorien

Man schmilzt ein Gemisch von 50 g 3S-Hydroxy-4S-BOC-Phe-His-amino-6-methyl-heptan-sulfonsäure-morpholid mit 10 g Sojalecithin und 140 g Kakaobutter, gießt in Formen und läßt erkalten. Jedes Suppositorium enthält 500 mg Wirkstoff.

## Ansprüche

1. Aminosäurederivate der Formel I

$$X-Z-NR^3-CHR^4-CR^5-(CHR^6)_n-E \qquad I$$

worin

X      H, $R^1-O-C_mH_{2m}-CO-$, $R^1-C_mH_{2m}-O-CO-$, $R^1-C_mH_{2m}-CO-$, $R^1-SO_2-$, $(R^1-C_mH_{2m})-L(R^2-C_pH_{2p})-C_rH_{2r}-CO-$, $R^1-(NHCH_2CH_2)_m-NH-CH_2CO-$, 9-Fluorenyl-$C_mH_{2m}-O-CO-$, $[R^1-C_mH_{2m}-(T)_x-V-C_tH_{2t}]-L-(R^2-C_pH_{2p})-C_rH_{2r}-CO-$oder $A_3N^{\oplus}-C_mH_{2m}-CO-$ $An^{\ominus}$,

Z      1 bis 4 peptidartig miteinander verbundene Aminosäurereste ausgewählt aus der Gruppe bestehend aus Abu, Ada, Ala, β-Ala, Arg, Asn, Asp, Bia, Cal, Dab, Ftr, Gln, Glu, Gly, His, Hph, N(im)-A-His, Ile, Leu, tert.-Leu, Lys, Met, αNal, βNal, Nbg, Nle, Nva, Orn, Phe, Pro, Pyr, Ser, Thr, Tia, Tic, Trp, Tyr, O-A-Tyr und Val,

E      $S-R^7$, $SO-R^7$, $SO_2-R^7$, $SO_2-OR^7$ oder $SO_2-NR^7R^8$,

    $R^1, R^2, R^4, R^7$ und $R^8$ jeweils H, A, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^3$    H oder A,

$R^5$    jeweils (H, OH), (H, NH₂) oder = O,

$R^6$    H, A oder Alkenyl mit 2-8 C-Atomen,

m, p, r und t    jeweils 0, 1, 2, 3, 4 oder 5,

n    1 oder 2,

x    0 oder 1,

L    CH oder N,

T    O oder NH,

V    CO oder $SO_2$,

$An^{\ominus}$    ein Äquivalent eines Anions,

Ar    unsubstituiertes oder ein- oder mehrfach durch A, AO, Hal, $CF_3$, HO, Hydroxyalkyl mit 1-8 C-Atomen, $H_2N$ und/oder Aminoalkyl mit 1-8 C-Atomen substituiertes Phenyl oder unsubstituiertes Naphthyl,

Het    einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, $CF_3$, HO, $O_2N$, Carbo-nylsauerstoff, $H_2N$, HAN, $A_2N$, AcNH, AS, ASO, $ASO_2$, AOOC, CN, $H_2NCO$, $H_2NSO_2$, $ASO_2NH$, Ar, Ar-alkenyl, Hy-droxyalkyl und/oder Aminoalkyl mit jeweils 1-8 C-Atomen substituiert sein kann und/oder dessen N- und/oder S-Heteroatome auch oxydiert sein können,

Hal    F, Cl, Br oder J,

Ac    A-CO-, Ar-CO- oder A-NH-CO- und

A    Alkyl mit 1-8 C-Atomen bedeuten,

worin ferner an Stelle einer oder mehrerer -NH-CO-Gruppen auch eine oder mehrere -NA-CO-Gruppen stehen können, sowie deren Salze.

2.

   a)   3S-Hydroxy-4S-BOC-Phe-His-amino-6-methyl-heptan-sulfonsäure-morpholid;

   b)   3S-Hydroxy-4S-[bis-(1-naphthylmethyl)-acetyl-His]-amino-6-methyl-heptan-sulfonsäuremorpholid;

   c)   1R-Isopropyl-3S-hydroxy-4S-BOC-Phe-His-amino-5-cyclohexyl-pentan-sulfonsäuremorpholid;

d)      1S-Isopropyl-3S-hydroxy-4S-BOC-Phe-His-amino-5-cyclohexyl-pentan-sulfonsäuremorpholid.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt
oder daß man eine Carbonsäure der Formel II

X-G$^1$-OH      II

worin G$^1$
(a) -Z$^1$-
(b) -Z- bedeutet und
X      die angegebene Bedeutung hat,
oder eines ihrer reaktionsfähigen Derivate
mit einer Verbindung der Formel III

H-G$^2$      III

worin
G$^2$
(a) -Z$^2$-NR$^3$-CHR$^4$-CR$^5$-(CHR$^6$)$_n$-E,
(b) -NR$^3$-CHR$^4$-CR$^5$-(CHR$^6$)$_n$-E und
Z$^1$ + Z$^2$      zusammen Z bedeuten und
R$^3$, R$^4$, R$^5$, R$^6$, n und E      die angegebenen
Bedeutungen haben,

oder einem ihrer reaktionsfähigen Derivate
umsetzt,
und/oder daß man gegebenenfalls zur Herstellung von Verbindungen der Formel I, worin E SO-R$^7$ oder -SO$_2$-R$^7$ bedeutet, ein Sulfid der Formel I, worin E S-R$^7$ bedeutet, mit einem Oxydationsmittel behandelt, und/oder daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine Ketogruppe zu einer CHOH-Gruppe reduziert oder zu einer CH(NH$_2$)-Gruppe reduktiv aminiert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.